# FASCICULE DE BREVET EUROPEEN

(11) **EP 2 986 277 B1**
(45) Date de publication et mention de la délivrance du brevet: **01.02.2017**
(21) Numéro de dépôt: 14719039.1
(22) Date de dépôt: 05.03.2014
(51) Int. Cl.: A61K 9/00, A61K 33/00, A61P 35/00, A61P 35/04, A61P 41/00

(54) **UTILISATION DE XÉNON POUR LIMITER LA MIGRATION DE CELLULES TUMORALES**
VERWENDUNG VON XENON ZUR BEGRENZUNG VON TUMORZELLENMIGRATION
USE OF XENON FOR LIMITING TUMOUR CELL MIGRATION

(30) Priorité: 15.04.2013 FR 1353394
(43) Date de publication de la demande: 24.02.2016
(73) Titulaire: L'Air Liquide Société Anonyme pour l'Etude et l'Exploitation des Procédés Georges Claude, 75007 Paris (FR)
(72) Inventeur: CHONG, Chui Fung, F-75015 Paris (FR); PYPE, Jan, B-1540 Herne (BE)
(74) Mandataire: Pittis, Olivier
(86) Numéro de dépôt international: PCT/FR2014/050488
(87) Numéro de publication internationale: WO 2014/170563

(56) Documents cités:
- EP-A1- 1 552 840
- EP-A1- 2 581 091
- TAVARE ANIKET N ET AL: "Cancer recurrence after surgery: Direct and indirect effects of anesthetic agents*", INTERNATIONAL JOURNAL OF CANCER, vol. 130, no. 6, mars 2012 (2012-03), pages 1237-1250, XP002716111,

## Description

L'invention concerne l'utilisation d'une composition gazeuse contenant du xénon gazeux pour prévenir ou pour limiter la migration des cellules tumorales et/ou à limiter ou à empêcher ainsi la formation de métastase chez un patient atteint d'un cancer, en particulier pour tuer tout ou partie des cellules cancéreuses véhiculées par la circulation sanguine chez ledit patient.

Le cancer est une maladie qui se caractérise par une prolifération cellulaire anormalement importante au sein d'un tissu ou organe normal de l'organisme et menaçant la survie de celui-ci. Les cellules cancéreuses dérivent toutes d'une même cellule initiale, à savoir la cellule initiatrice du cancer, qui a acquis certaines caractéristiques lui permettant de se diviser indéfiniment.

Au cours de l'évolution de la maladie, certaines cellules sont libérées ou se détachent du tissu ou organe cancéreux, et peuvent migrer de leur lieu de production vers d'autres endroits de l'organisme. Ces cellules libres et migrantes conduisent donc à la naissance d'autres foyers cancéreux dans l'organisme, appelés métastases

Ces cellules migrantes sont libérées soit spontanément, soit inopinément lors d'une intervention chirurgicale ayant lieu sur le patient.

En effet, une des techniques chirurgicales les plus répandues pour éliminer les cancers est basée sur une suppression du foyer cancéreux par chirurgie.

Pour ce faire, on découpe, par scalpel, faisceau laser ou autre, les tissus cancéreux ainsi qu'une partie des tissus non encore infectés sur lesquels se sont développés lesdits tissus cancéreux. On élimine ainsi toute la tumeur cancéreuse mais aussi des tissus sains pour ne pas prendre le risque de couper directement dans la tumeur et de disséminer ainsi un grand nombre de cellules cancéreuses qui pourraient se retrouver dans les liquides corporels, en particulier sang et lymphe.

Or, si cette technique de découpe des tissus cancéreux est radicale en ce qu'elle permet de supprimer la totalité des tissus cancéreux formant le foyer cancéreux, elle présente l'inconvénient de conduire malgré tout à une libération de cellules cancéreuses migrantes qui peuvent alors être entraînées ailleurs dans l'organisme en étant véhiculées par le sang et former des métastases ailleurs dans le corps en infectant d'autres tissus ou organes.

Pour tenter d'y remédier, certaines substances anticancéreuses sont parfois utilisées, dans le cadre d'une chimiothérapie, de manière à lutter contre la formation de métastases mais ces substances chimiques engendrent souvent des effets secondaires indésirables chez les patients, par exemple des douleurs neuropathiques.

Le problème qui se pose est dès lors de pouvoir disposer d'une composition ou d'un médicament efficace pour prévenir, limiter, ralentir ou freiner la migration des cellules tumorales de manière à limiter ou à empêcher formation de métastases chez un individu atteint d'un cancer, en particulier une composition ou un médicament capable de tuer ou éliminer tout ou partie des cellules cancéreuses libres véhiculées par le sang d'un individu, typiquement un patient humain, notamment les cellules cancéreuses libérées lors d'une intervention de chirurgie visant à extraire un foyer cancéreux chez ledit individu, et ce, sans engendrer d'effets secondaires chez celui-ci.

La solution selon l'invention est une composition gazeuse contenant du xénon en tant que principe actif pour une utilisation pour prévenir ou pour limiter la migration des cellules tumorales et/ou limiter ou empêcher la formation de métastases, chez un individu atteint d'un cancer.

EP 1552840 décrit des compositions gazeuses contenant un mélange de xénon et de monoxyde de carbone pour inhiber la prolifération indésirable de cellules, notamment de cellules tumorales. Cependant, ce document ne décrit aucun effet sur la migration des cellules cancéreuses ou la formation de métastases. Dans le cadre de l'invention, on précise que :
- par « patient », on entend un individu atteint d'un cancer, que ce soit un homme, une femme, un enfant, un nouveau né...
- par « cellule cancéreuse libre », on entend une cellule non rattachée à un tissu ou organe cancéreux (i.e. un foyer cancéreux) mais provenant d'un tel foyer cancéreux et susceptible d'être véhiculée.
- par « % », on entend un pourcentage volumique, i.e. % en volume, sauf indication différente.
- les termes « prévenir », « limiter », « ralentir » ou « freiner » sont considérés comme sensiblement équivalents pour signifier schématiquement « empêcher » ou « lutter contre ».

Selon le cas, la composition gazeuse de l'invention utilisée en tant que médicament inhalable peut comprendre l'une ou plusieurs des caractéristiques techniques suivantes :
- elle est administrable par inhalation, par exemple au moyen d'un masque respiratoire.
- elle est administrable par insufflation, par exemple au moyen d'un endoscope, d'un tuyau adapté à cet usage ou analogue.
- la prévention ou limitation de formation de métastase est obtenue par action du xénon sur lesdites cellules cancéreuses libres.
- le xénon agit sur les cellules cancéreuses libres véhiculées par le sang.
- le xénon agit sur les cellules cancéreuses libres en bloquant l'action métastasique d'au moins une partie desdites cellules cancéreuses libres.
- le xénon est administré pendant que le patient subit une intervention de chirurgie visant à extraire le foyer cancéreux chez ledit patient.
- l'administration de xénon débute avant pendant une intervention de chirurgie visant à extraire le foyer cancéreux chez ledit patient, se poursuit pendant l'intervention de chirurgie, de préférence l'administration de xénon se prolonge après la fin d'intervention.
- le xénon agit sur les cellules cancéreuses libres véhiculées par le sang qui sont libérées pendant une intervention de chirurgie visant à extraire un foyer cancéreux chez ledit patient.
- le patient est atteint d'un cancer dit « solide » choisi parmi les cancers du foie, des reins, du cerveau, de la peau, du sein ou colorectal.
- la proportion volumique efficace de xénon est comprise entre 5 et 79%.
- la proportion volumique efficace de xénon est comprise entre 5 et 75%.
- la proportion volumique efficace de xénon est comprise entre 5 et 70%.
- la proportion volumique de xénon est d'au moins 10%.
- la proportion volumique de xénon est d'au moins 20%.
- la proportion volumique de xénon est d'au moins 30%.
- la proportion volumique de xénon est d'au moins 40%.
- la proportion volumique de xénon est d'au moins 50%.
- la proportion volumique de xénon est d'au moins 60%.
- la proportion volumique de xénon est d'au moins 65%.
- la proportion volumique de xénon est comprise entre 62 et 75%.
- la proportion volumique de xénon est comprise entre 65 et 73%.
- elle contient en outre une proportion volumique d'oxygène (O₂) d'au moins 21%, typiquement entre 21% et 50%, en particulier entre 21 et 35% d'oxygène.
- elle est administrée par inhalation pendant au moins 15 minutes par jour, de préférence au moins 30 min/jour, de préférence encore au moins 1 h/jour.
- elle est administrée par inhalation pendant une durée suffisante pour obtenir un effet observable dans le ou les jours suivant son administration par inhalation.
- elle est administrée par inhalation de manière continue ou fractionnée, c'est-à-dire à plusieurs reprises ou en plusieurs fois.
- optionnellement, elle est co-administrée avec une substance de chimiothérapie anticancéreuse, c'est-à-dire administrée avant, pendant et/ou après l'administration de la substance de chimiothérapie anticancéreuse.
- elle est administrée par inhalation pendant au moins toute la période de temps de traitement du patient par la substance de chimiothérapie anticancéreuse. Ainsi, si le patient doit prendre la substance de chimiothérapie anticancéreuse pendant 6 mois par exemple, alors le médicament est administré également pendant cette même durée de 6 mois, voire un peu plus longtemps pour pallier à des éventuels effets retards.
- elle contient, en outre, un gaz additionnel choisi parmi l'argon, l'hélium, le néon, le krypton, le NO, le CO, le sulfure d'hydrogène (H₂S) et l'azote, y compris de l'air.
- le patient est un être humain, c'est-à-dire un homme ou une femme, y compris les enfants, les adolescents ou tout autre groupe d'individus, par exemple les nouveau-nés ou les personnes âgées.
- il est prêt à l'emploi.
- il est conditionné dans une bouteille de gaz, en particulier une bouteille ou bonbonne de gaz équipée d'un robinet ou d'un robinet à détendeur intégré. De préférence, le robinet ou robinet à détendeur intégré est protégé par un capotage de protection.

En d'autres termes, selon la présente invention, on propose d'administrer par inhalation ou insufflation, une composition à base de xénon, de préférence contenant plus de 60% de xénon et le reste d'oxygène, afin de prévenir ou de limiter la du xénon en tant que principe actif pour une utilisation pour prévenir ou pour limiter la migration des cellules tumorales de manière à limiter ou à empêcher formation de métastase chez un individu atteint d'un cancer, notamment en vue de tuer tout ou partie des cellules cancéreuses susceptibles d'avoir été libérées durant une opération chirurgicale visant à supprimer un foyer cancéreux.

La concentration et/ou la durée d'administration la plus adaptée à un patient donné peut être choisie(s) de manière empirique par le personnel soignant, par exemple en fonction de l'état de santé ou physique du patient, de la sévérité de la douleur, de son sexe, de son âge, du type de cancer, du type d'agent anticancéreux co-administré, etc...

Le gaz à base de xénon selon l'invention peut être mis en oeuvre dans le cadre d'une méthode de traitement thérapeutique, dans laquelle on administre le mélange gazeux, par exemple par inhalation au moyen d'un masque respiratoire soit directement relié à une source de xénon à la concentration requise, par exemple une bouteille de gaz prêt à l'emploi, ou alors à la sortie d'un mélangeur de gaz alimenté par plusieurs sources de gaz (O₂, Xe...) de manière à obtenir le mélange souhaité ; soit relié à un ventilateur respiratoire alimenté en le ou les gaz souhaités.

Dit autrement, la présente invention est donc basée sur l'utilisation d'un mélange gazeux thérapeutique contenant du xénon en tant que produit actif en une proportion efficace pour fabriquer un médicament inhalable à effets curatifs destiné à limiter la migration des cellules tumorales, à savoir des cellules cancéreuse libres et véhiculées par le sang d'un patient atteint d'un cancer et ayant subi une intervention chirurgicale visant à cureter les tissus ou l'organe affectés par ledit cancer.

La présente invention va maintenant être mieux comprise grâce à la description suivante et aux exemples qui vont suivre, qui sont donnés à titre purement illustratif et en références aux Figures annexées parmi lesquelles :
- La Figure 1 représente la migration de cellules cancéreuses ER- de type MDA-MB-231 exposées ou non à une atmosphère contenant du xénon gazeux.
- La Figure 2 représente la migration de cellules cancéreuses ER+ de type MCF7 exposées ou non à une atmosphère contenant du xénon gazeux.

Afin de démontrer l'efficacité du gaz à base de xénon selon l'invention dans la lutte contre la migration des cellules cancéreuses libres véhiculées par le sang d'un individu cancéreux, des essais ont été réalisés dans les conditions suivantes.

Des cellules cancéreuses ER- et ER+ (Estrogen Receptor = Récepteur à OEstrogène) d'un adénocarcinome humain (cancer du sein), à savoir des lignées de cellules MDA-MB-231 et MCF7, ont été déposées sur des plaques revêtues de collagène où elles ont adhéré. Ces lignées cellulaires sont disponibles commercialement.

Les plaques avec cellules adhérentes ont été introduites ensuite dans une chambre hermétique, saturée, pendant une durée de 1 h, 3 h et 5 h, avec :
- soit une atmosphère de contrôle (témoin), à savoir un mélange gazeux formé de 25% de O₂, 5% de CO₂ et 70% de N₂ (% en volume) ;
- soit une atmosphère de test à base de xénon selon l'invention, à savoir un mélange gazeux formé de 25% de O₂, 5% de CO₂ et 70% xénon (% en volume).

La survie cellulaire a été déterminée après 24 heures, en utilisant un test MTT (pour methylthaizolyldiphenyl-tetrazolium-bromide). Ce test est un test classique permettant de déterminer la viabilité cellulaire. Il est basé sur une détection uniquement des cellules vivantes, via l'activité de leurs déshydrogénases mitochondriales, ce qui permet d'évaluer la cytotoxicité, la prolifiération et/ou l'activation cellulaire.

La migration cellulaire à 24 h a, quant à elle, été déterminée après 24 heures, en utilisant des cellules marquées par fluorescence dans le cadre d'un test Oris™ de migration cellulaire. Dans ce test, les cellules marquées migrent dans une zone cible et y sont alors quantifiées par un lecteur de plaques fluorescentes permettant de mesurer les unités relatives fluorescentes (i.e. relative fluorescent unit ou RFU)).

Ces tests ont montré que le xénon n'affecte pas significativement la survie cellulaire.

Par contre, le xénon inhibe la migration cellulaire à la fois chez les cellules MDA-MB-231 et les cellules MCF7 par rapport au groupe témoin (cellules non mises en contact avec du xénon) et ce, dans les proportions du Tableau suivant et ce, quel que soit le temps d'exposition au gaz.

**Tableau**

| | % de migration cellulaire par rapport au Témoin | | |
|---|---|---|---|
| Temps d'exposition xénon (heures) | 1 h | 3 h | 5 h |
| Cellules | | | |
| MDA-MB-231 | 59 ± 13% | 64 ±10% | 71 ± 9% |
| | (n=4, P=0.02) | (n=4; P=0.01) | n=3, P=0.04 |
| Cellules | | | |
| MCF7 | 58±12% | 65±12% | 65±12% |
| | n=4, *P*=0.01 | n=4, P=0.03 | n=3, P=0.04 |

| | | | |
|---|---|---|---|
| n : nombre de tests/essais P = valeur de probabilité (significativité avérée si p<0.05) | | | |

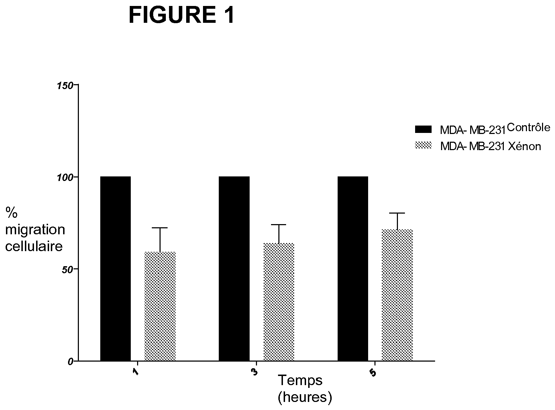

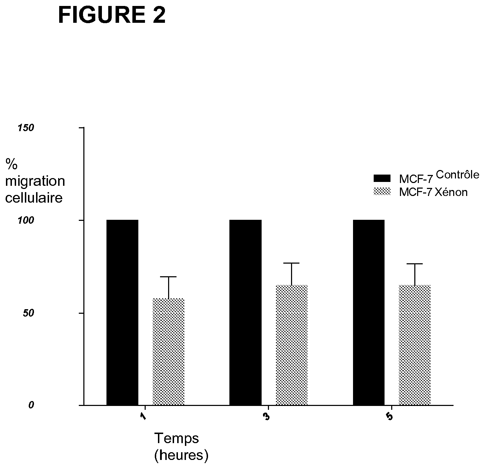

Ces résultats sont illustrés sur les Figures 1 et 2.

Plus précisément, comme déjà dit, plusieurs tests (3 ou 4 tests) ont été opérés avec les différentes atmosphères gazeuses pendant différentes durées d'exposition, et les résultats ont été exprimés sous forme d'unités fluorescentes relatives (i.e. relative fluorescent units = RFU).

La migration des cellules cancéreuses exposées à l'atmosphère contenant du xénon a été calculée en pourcentage (%) par rapport à celle des cellules exposées à l'atmosphère témoin, c'est-à-dire exposée à une atmosphère sans xénon (RFU fixée à 100%), et dans les mêmes conditions.

Un test t-paramétrique bilatéral pour analyser statistiquement la différence entre les lignées cellulaires et les valeurs de probabilité P inférieure à 0,05 ont été considérées significatives.

Comme on le constate sur la Figure 1, le xénon (Xe) réduit significativement la migration des cellules ER- MDA-MB-231, ainsi que celle des cellules ER+ MCF7, comme illustré en Figure 2, et ce, dès 1 heure d'exposition.

Ceci montre l'efficacité du xénon gazeux pour prévenir ou pour limiter la migration des cellules tumorales de manière à limiter ou à empêcher formation de métastase chez un individu atteint d'un cancer.

## Revendications

1. Composition gazeuse contenant du xénon en tant que principe actif pour une utilisation pour prévenir ou pour limiter une migration des cellules tumorales et/ou à limiter ou à empêcher une formation de métastase chez un individu atteint d'un cancer.

2. Composition pour utilisation selon la revendication précédente, **caractérisée en ce que** la prévention ou limitation de la migration des cellules tumorales est obtenue par action du xénon sur les cellules cancéreuses libres.

3. Composition pour utilisation selon l'une des revendications précédentes, **caractérisée en ce que** le xénon agit sur les cellules cancéreuses libres véhiculées par le sang.

4. Composition pour utilisation selon l'une des revendications précédentes, **caractérisée en ce que** le xénon agit sur les cellules cancéreuses libres en bloquant l'action métastasique d'au moins une partie desdites cellules cancéreuses libres

5. Composition pour utilisation selon l'une des revendications précédentes, **caractérisée en ce que** la proportion volumique efficace de xénon est comprise entre 5 et 79%.

6. Composition pour utilisation selon l'une des revendications précédentes, **caractérisée en ce que** la proportion volumique efficace de xénon est d'au moins 50%.

7. Composition pour utilisation selon l'une des revendications précédentes, **caractérisée en ce qu'**elle contient en outre une proportion volumique d'oxygène (O₂) d'au moins 21%.

8. Composition pour utilisation selon l'une des revendications précédentes, **caractérisée en ce qu'**elle contient, en outre, un gaz additionnel choisi parmi l'air, l'argon, l'hélium, le néon, le krypton, le NO, le CO, le sulfure d'hydrogène (H₂S), le N₂O et l'azote.

9. Composition pour utilisation selon l'une des revendications précédentes, **caractérisée en ce qu'**elle est co-administrée avec une substance de chimiothérapie anticancéreuse.

10. Composition pour utilisation selon l'une des revendications précédentes, **caractérisée en ce que** l'individu est un être humain.

11. Composition pour utilisation selon l'une des revendications précédentes, **caractérisée en ce qu'**elle est administrable par inhalation ou par insufflation.

12. Composition pour utilisation selon l'une des revendications précédentes, **caractérisée en ce que** le patient est atteint d'un cancer du foie, du rein, du cerveau, de la peau, du sein ou colorectal.

13. Composition pour utilisation selon l'une des revendications précédentes, **caractérisée en ce que** le xénon est administré pendant que l'individu subit une intervention de chirurgie visant à extraire le foyer cancéreux chez ledit individu.

14. Composition pour utilisation selon l'une des revendications précédentes, **caractérisée en ce que** l'administration de xénon débute avant pendant une intervention de chirurgie visant à extraire le foyer cancéreux chez ledit individu, se poursuit pendant l'intervention de chirurgie, de préférence l'administration de xénon se prolonge après la fin d'intervention.

15. Composition pour utilisation selon l'une des revendications précédentes, **caractérisée en ce que** le xénon agit sur les cellules cancéreuses libres véhiculées par le sang qui sont libérées pendant une intervention de chirurgie visant à extraire un foyer cancéreux chez ledit individu.

## Patentansprüche

1. Gaszusammensetzung, welche Xenon als Wirkstoff enthält, zur Verwendung um eine Migration von Tumorzellen zu vermeiden oder zu begrenzen und/oder die Bildung von Metastasen bei einem an Krebs erkrankten Individuum zu begrenzen oder zu verhindern.

2. Zusammensetzung zur Verwendung
nach dem vorstehenden Anspruch, **dadurch gekennzeichnet, dass** die Vermeidung oder Begrenzung der Migration der Tumorzellen durch Wirkung des Xenons auf die freien Krebszellen erzielt wird.

3. Zusammensetzung zur Verwendung
nach einem der vorstehenden Ansprüche, **dadurch gekennzeichnet, dass** das Xenon auf die vom Blut getragenen freien Krebszellen wirkt.

4. Zusammensetzung zur Verwendung
nach einem der vorstehenden Ansprüche, **dadurch gekennzeichnet, dass** das Xenon auf die freien Krebszellen wirkt, indem es die metastasierende Tätigkeit von zumindest einem Teil der freien Krebszellen blockiert.

5. Zusammensetzung zur Verwendung
nach einem der vorstehenden Ansprüche, **dadurch gekennzeichnet, dass** der effektive Volumenanteil an Xenon zwischen 5 und 79 % beträgt.

6. Zusammensetzung zur Verwendung
nach einem der vorstehenden Ansprüche, **dadurch gekennzeichnet, dass** der effektive Volumenanteil an Xenon mindestens 50 % beträgt.

7. Zusammensetzung zur Verwendung
nach einem der vorstehenden Ansprüche, **dadurch gekennzeichnet, dass** sie des Weiteren einen Volumenanteil an Sauerstoff (O₂) von mindestens 21 % enthält.

8. Zusammensetzung zur Verwendung
nach einem der vorstehenden Ansprüche, **dadurch gekennzeichnet, dass** sie des Weiteren ein zusätzliches Gas enthält, ausgewählt aus Luft, Argon, Helium, Neon, Krypton, NO, CO, Schwefelwasserstoff (H₂S), N₂O und Stickstoff.

9. Zusammensetzung zur Verwendung
nach einem der vorstehenden Ansprüche, **dadurch gekennzeichnet, dass** sie gleichzeitig mit einem chemotherapeutischen Antikrebsmittel verabreicht wird.

10. Zusammensetzung zur Verwendung
nach einem der vorstehenden Ansprüche, **dadurch gekennzeichnet, dass** es sich bei dem Individuum um einen Menschen handelt.

11. Zusammensetzung zur Verwendung
nach einem der vorstehenden Ansprüche, **dadurch gekennzeichnet, dass** sie durch Einatmen oder durch Einblasen verabreicht werden kann.

12. Zusammensetzung zur Verwendung
nach einem der vorstehenden Ansprüche, **dadurch gekennzeichnet, dass** der Patient an einem Leber-, Nieren-, Gehirn-, Haut-, Brust- oder Darmkrebs erkrankt ist.

13. Zusammensetzung zur Verwendung
nach einem der vorstehenden Ansprüche, **dadurch gekennzeichnet, dass** das Xenon verabreicht wird, während das Individuum einem chirurgischen Eingriff unterzogen wird, welcher das Ziel hat, den Krebsherd bei dem Individuum zu entfernen.

14. Zusammensetzung zur Verwendung
nach einem der vorstehenden Ansprüche, **dadurch gekennzeichnet, dass** die Verabreichung von Xenon vor während einem chirurgischen Eingriff beginnt, welcher das Ziel hat, den Krebsherd bei dem Individuum zu entfernen, während des chirurgischen Eingriffs andauert, bevorzugt die Verabreichung von Xenon nach dem Ende des Eingriffs fortgesetzt wird.

15. Zusammensetzung zur Verwendung
nach einem der vorstehenden Ansprüche, **dadurch gekennzeichnet, dass** das Xenon auf die vom Blut getragenen freien Krebszellen wirkt, die während eines chirurgischen Eingriffs, welcher das Ziel hat, einen Krebsherd bei dem Individuum zu entfernen, freigesetzt werden.

## Claims

1. Gaseous composition containing xenon as an active principle for use for preventing or limiting migration of tumour cells and/or limiting or preventing formation of metastasis in an individual suffering from cancer.

2. Composition for use according to the preceding claim, **characterised in that** the prevention or limitation of the migration of tumour cells is obtained by the action of xenon on the free cancerous cells.

3. Composition for use according to one of the preceding claims, **characterised in that** the xenon acts on the free cancerous cells conveyed by the blood.

4. Composition for use according to one of the preceding claims, **characterised in that** the xenon acts on the free cancerous cells by blocking the metastatic action of at least some of said free cancerous cells.

5. Composition for use according to one of the preceding claims, **characterised in that** the effective proportion by volume of the xenon is between 5% and 79%.

6. Composition for use according to one of the preceding claims, **characterised in that** the effective proportion by volume of the xenon is at least 50%.

7. Composition for use according to one of the preceding claims, **characterised in that** it further contains a proportion by volume of oxygen (O₂) of at least 21%.

8. Composition for use according to one of the preceding claims, **characterised in that** it further contains an additional gas chosen from air, argon, helium, neon, krypton, NO, CO, hydrogen sulphide (H₂S), N₂O and nitrogen.

9. Composition for use according to one of the preceding claims, **characterised in that** it is co-administered with an anti-cancer chemotherapy substance.

10. Composition for use according to one of the preceding claims, **characterised in that** the individual is a human being.

11. Composition for use according to one of the preceding claims, **characterised in that** it can be administered by inhalation or insufflation.

12. Composition for use according to one of the preceding claims, **characterised in that** the patient is suffering from liver, renal, brain, skin, breast or colorectal cancer.

13. Composition for use according to one of the preceding claims, **characterised in that** the xenon is administered while the individual is undergoing surgery aimed at extracting the malignant focus in said individual.

14. Composition for use according to one of the preceding claims, **characterised in that** the administration of the xenon begins before during surgery aimed at extracting the malignant focus in said individual, continues during the surgery, and preferably the administration of xenon is extended after the end of surgery.

15. Composition for use according to one of the preceding claims, **characterised in that** the xenon acts on the free cancerous cells conveyed by the blood that are released during surgery aimed at extracting a malignant focus in said individual.
